(19) Europäisches Patentamt
European Patent Office
Office européen des brevets

(11) Veröffentlichungsnummer: 0 264 051
A2

(12) EUROPÄISCHE PATENTANMELDUNG

(21) Anmeldenummer: 87114554.6

(22) Anmeldetag: 06.10.87

(51) Int. Cl.4: C07C 69/94 , C07C 65/105 , B41M 5/00

(30) Priorität: 17.10.86 DE 3635311

(43) Veröffentlichungstag der Anmeldung: 20.04.88 Patentblatt 88/16

(84) Benannte Vertragsstaaten: BE DE FR GB IT NL

(71) Anmelder: BAYER AG
Konzernverwaltung RP Patentabteilung
D-5090 Leverkusen 1 Bayerwerk(DE)

(72) Erfinder: Botta, Artur, Dr.
Bodelschwinghstrasse 119
D-4150 Krefeld(DE)
Erfinder: Jabs, Gert, Dr.
Wingensiefer Kamp 25
D-5068 Odenthal-Glöbusch(DE)

(54) **Hydroxycarbonsäurederivate, Verfahren zu ihrer Herstellung und ihre Verwendung in druck- oder wärmeempfindlichen Aufzeichnungsmaterialien.**

(57) Kondensationsprodukte aus Benzylchoriden und aromatischen Hydroxycarbonsäuren eignen sich als Farbentwickler in druck-oder wärmeempfindlichen Aufzeichnungsmaterialien.

EP 0 264 051 A2

# Hydroxycarbonsäurederivate, Verfahren zu ihrer Herstellung und ihre Verwendung in druck-oder wärmeempfindlichen Aufzeichnungsmaterialien

Die vorliegende Erfindung betrifft Hydroxycarbonsäurederivate und ihre Verwendung in druck-oder wärmeempfindlichen Aufzeichnungsmaterialien.

Kondensationsprodukte aus aromatischen Hydroxycarbonsäuren und Benzylhalogeniden sind bereits bekannt. Nach dem DRP 564 127 werden solche durch Erhitzen von aromatischen Hydroxycarbonsäuren mit stark überschüssigen Mengen von Benzylhalogeniden in der Regel in Gegenwart geringer katalytischer Mengen wasserfreier Kondensationsmittel wie $ZnCl_2$ bei Temperaturen bis über 200 → 250°C hergestellt. Es handelt sich um harzartige komplexe Gemische mit geringer Säurezahl, insbesondere auch dadurch bedingt, daß bei den hohen Reaktionstemperaturen bereits eine weitgehende Decarboxylierung der eingesetzten Hydroxycarbonsäuren stattfindet. Da Benzylchloride bekanntlich in Gegenwart von Lewissäuren eine Selbstkondensation zu Polybenzylharzen eingehen, liegt nach der Verfahrensweise der obengenannten Patentschrift ein erheblicher Anteil an Selbstkondensationsharz neben Cokondensationsharz vor.

Nach der bekannten Verfahrensweise hergestellte Kondensationsprodukte aus Salicylsäure und Benzylchlorid stellen dunkelrotbraune Harze dar.

Weiterhin sind nach dem DRP 346 384 Kondensationsprodukte aus Phenolen und Benzylhalogeniden bekannt, die in Abwesenheit von speziellen Kondensationsmitteln hergestellt werden. Im Falle der gegenüber den reinen Phenolen weniger reaktiven Phenolcarbonsäuren, wie Salicylsäure, ist die Umsetzung jedoch selbst bei Einsatz von nur 2 Mol Benzylchlorid pro Mol Salicylsäure nicht vollständig, wie Beispiele 3 und 5 zeigen.

Die genannten Kondensationsmassen werden zur Herstellung von Öllacken vorgeschlagen. Über eine Verwendung zur Herstellung von Farbentwicklern für druck-und wärmeempfindliche Aufzeichnungsmaterialien ist nichts bekannt. Sie sind auf Grund der starken Eigenfärbung auch für diesen Anwendungszweck nicht geeignet.

Aus der EP-A 181 283 und JP 5 820 779 sind bereits Metallsalicylate und ihre Verwendung als Farbentwickler in Aufzeichnungsmaterialien bekannt. Die Herstellung dieser Materialien ist relativ aufwendig, da in Lösung gearbeitet werden muß. Außerdem fallen die Substanzen kristallin an und haben hohe Schmelzpunkte. Besonders bei druckempfindlichen Aufzeichnungssystemen hängt die Schnelligkeit und Intensität der Farbentwicklung jedoch davon ab, wie schnell und gut das Farbgebelösungsmittel den Farbentwickler anlösen kann. Nichtkristalline Harze mit breiter Molekulargewichtsverteilung und deshalb niedrigerem Erweichungspunkt sind in dieser Hinsicht von deutlichem Vorteil.

Unter Aufzeichnungsmaterialien werden für die Zwecke der vorligenden Erfindung insbesondere Materialien verstanden, auf denen durch bildmäßigen mechanischen Druck oder durch bildmäßiges Erhitzen sichtbare Darstellungen erzeugt werden können.

Hierzu gehören die bekannten Reaktionsdurchschreibepapiere (vgl. M. Gutcho, Capsule Technology and Mikroencapsulation, Noyes Data Corporation, 1972, Seiten 242-277; G. Baxter in Microencapsulation, Processes and Applications, herausgegeben von J.E. Vandegaer, Plenum Press New York, London, Seiten 127-143).

Reaktionsdurchschreibepapiere bestehen beispielsweise aus zwei oder mehreren lose aufeinandergelegten Papierblättern, wobei das jeweils obere auf der Rückseite eine Geberschicht und das jeweils untere auf der Vorderseite eine Nehmerschicht enthält. Es ist also jeweils eine Geberschicht und eine Nehmerschicht miteinander in Kontakt. Die Geberschicht enthält z.B. Mikrokapseln, deren Kernmaterial eine Lösung eines Farbstoffbildners in einem organischen Lösungsmittel ist und die Nehmer schicht enthält einen Farbentwickler, d.h. ein Material, das den Farbstoffbildner zu Farbstoff umwandelt. Eine Durchschrift entsteht, wenn die Mikrokapseln durch den Druck eines Schreibgerätes zerstört werden und der Farbstoffbildner mit dem Farbentwickler bildmäßig reagiert.

Wenn der Farbstoffbildner in einem schmelzbaren Wachs eingebettet ist statt in Mikrokapseln, entsteht eine Durchschrift, wenn das Papier bildmäßig erhitzt wird. Es handelt sich dann um ein thermoreaktives Aufzeichnungssystem.

Farbstoffbildner und Farbentwickler können auch auf demselben Papierblatt aufgebracht sein. Man spricht dann von "self contained paper". Auf solchem Material kann durch bildmäßigen Druck bzw. bildmäßiges Erhitzen z.B. eine Schrift erzeugt werden.

Thermoreaktive Aufzeichnungssysteme werden bevorzugt in elektronischen Rechnern, Fettdruckern, Fernschreibern und Meßinstrumenten verwendet. Man kann auf ihnen Markierungen auch mit Hilfe von Laserstrahlen erzeugen.

Thermoreaktive Aufzeichnungssystem können so aufgebaut sein, daß der Farbstoffbildner in einer Bindemittelschicht gelöst oder dispergiert ist und in einer zweiten Schicht der Farbentwickler in demselben Bindemittel gelöst oder dispergiert ist. Farbstoffbildner und Farbentwickler können aber auch in einer Schicht dispergiert werden. Der Farbentwickler wird durch Wärme erweicht und kommt an den Stellen, an denen Wärme angewendet wird, mit dem Farbgeber in Kontakt. Dabei entwickelt sich die Farbe.

Typische Beispiele für bekannte Farbentwickler sind Aktivton-Substanzen, wie Attapulgus-Ton, Säureton, Bentonit oer Montmorillonit; ferner Halloysit, Zeolith, Siliciumdioxid, Aluminiumoxid, Aluminiumsulfat, Aluminiumphosphat, Zinkchlorid, Kaolin sowie andere Tone oder sauer reagierende organische Verbindungen, z.B. ringsubstituierte Phenole, Salicylsäure, deren Metallsalze oder Salicylsäureester, ferner sauer reagierende polymere Materialien, wie phenolische Polymerisate, Alkylphenolacetylenharze, Maleinsäure-kolophonium-Harz oder teilsweise oder vollständig hydrolysierte Polymerisate aus Maleinsäureanhydrid und Styrol, Ethylen oder Vinylmethylether oder Polyacetale.

Die Farbentwickler können zusätzlich auch im Gemisch mit an sich unreaktiven oder wenig reaktiven Pigmenten oder weiteren Hilfsstoffen wie Kieselgel eingesetzt werden. Beispiele für solche Pigmente sind: Talk, Titandioxid, Zinkoxid, Kreide, Tone wie Kaolin sowie organische Pigmente, z.B. Harnstoff-Formaldehyd-oder Melamin-Formaldehyd-Kondensationsprodukte.

Aktivierte Tone sind feuchtigkeitsempfindlich, d.h. entwickelte Farbe läßt sich mit Wasser beseitigen oder bildet sich in einer feuchten Atmospäre nur sehr schwach aus. Die Entwicklung schwarzer Fluoranfarbstoffe, wie z.B. 3-Diethylamino-6-methyl-7-anilinofluoran oder 3-(N-Cyclohexyl-N-methylamino)-6-methyl-7-anilinofluoran ist nicht möglich. Vielmehr ergeben sich mit entsprechenden organischen Farbbildnern rötlich-schwarze oder grünlich-schwarze Bilder, die schnell zu rotbraunen verblassen.

In der Deutschen Auslegeschrift 22 42 250 sowie den Belgischen Patentschriften 784 913 und 802 914 werden Metallsalze von substituierten Hydroxyarylcarbonsäuren, z.B. der Salicylsäure als Farbentwickler beschrieben. Aus der DE-A-2 348 639 sind außerdem Mischungen aus aromatischen Carbonsäuren oder von deren Salzen mit Polymeren bekannt. Die aromatischen Carbonsäuren bzw. deren Salze sind häufig in Wasser löslich, weshalb beim Auftragen der wäßrigen Druckpaste ein Teil der Säuren in das Innere des Blattes diffundiert, woraus ein geringeres Farbbildungsvermögen und damit eine niedrigere Farbdichte resultiert.

Phenole als Farbentwickler werden z.B. in US-A-3 244 550 beschrieben, Phenolharze z.B. in US-A-3 672 935 und schließlich der spezielle Einsatz von Bisphenol-A-Harzen, z.B. in JA-PS 063 958.

Die bekannten Phenole und Phenolharze weisen insbesondere folgende Nachteile auf:

-Bei der Durchschrift zeigt sich - etwa im Vergleich zu Ton-Entwicklern - eine geringere Intensität bzw. Farbstärke.

-Die Entwicklungsgeschwindigkeit der Durchschrift ist gering. Zu Beginn bleibt die Durchschrift blaß, bis sich im Laufe der Zeit allmählich die Intensität erhöht.

-Beschichtete Farbenwickler-Papiere auf Phenolharzbasis zeigen eine ausgeprägte Vergilbungstendenz. Diese wird durch Sonnenlicht, aber auch künstliche Lichtquellen noch verstärkt.

-Die Herstellung einer wäßrigen Phenolharz-Disperison zur Beschichtung ist häufig sehr schwierig, weil die Harze wegen ihrer hohen Schmelztemperatur, ihrer hohen Schmelzviskosität und ihrer großen Verklebungsneigung nur sehr schwierig homogen dispergierbar sind.

-Bei Phenol-Formaldehydharzen besteht die Gefahr einer Rückspaltung zu Phenol und Formaldehyd. Formaldehyd ist aus bekannten Gründen nicht unbedenklich.

Gegenstand der Erfindung sind Verbindungen folgender Formel I

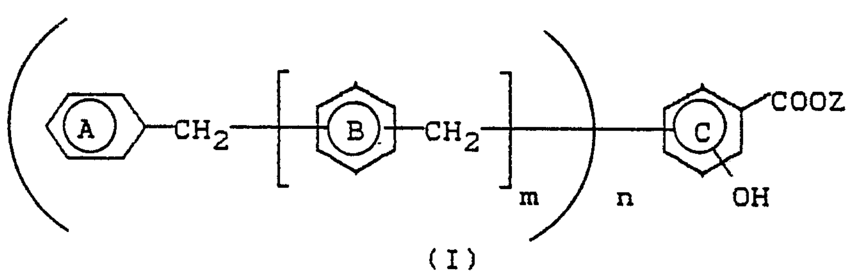

(I)

worin bedeuten

Z Wasserstoff, Alkyl mit 1-6 C-Atomen oder

$$\frac{M^{z+}}{z}$$

M z-wertiges Metallkation, insbesondere der 2. oder 3. Hauptgruppe oder ein Übergangsmetallkation
z 1 bis 4, bevorzugt 2 und 3
n 1 bis 4
m 0 bis 20, insbesondere 0 - 10
mit der Maßgabe, daß m + n wenigstens 2 bedeuten, wobei die Ringe A, B und C unabhängig voneinander unsubstituiert sein können oder substituiert sein können, insbesondere mit Alkyl, Cycloalkyl, Aryl, Halogen, Alkoxy, Aryloxy, OH, Carboxy, Alkoxycarbonyl oder einer Nitrogruppe und wobei die Verbindungen auch bimolekular vorliegen können indem ein Substituent 2 Reste der Formel I miteinander verknüpft. In einer anderen besonders bevorzugten Ausführungsform sind die Ringe nicht substituiert.

Gegenstand der Erfindung sind weiterhin Harze, die im wesentlichen Verbindungen der Formel (I) aufweisen. Bevorzugte Verbindungen der allgemeinen Formel (I) entsprechen folgender Formel (II)

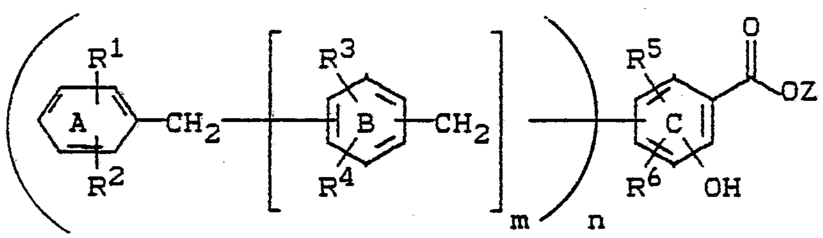

(II)

worin bedeuten
$R^1,R^2,R^3,R^4$: gleich oder verschieden, Wasserstoff, Alkyl mit 1 bis 6 C-Atomen, Cycloalkyl, insbesondere mit 5 oder 6 C-Atomen, Aralkyl, insbesondere mit 1 bis 4 C-Atomen im aliphatischen Teil, Aryl, insbesondere Phenyl, Halogen, Alkoxy mit 1 bis 4 C-Atomen, Phenoxy oder Nitro,
$R^5$ und $R^6$, gleich oder verschieden, Wasserstoff, Alkyl, mit 1 bis 4 C-Atomen, Cycloalkyl, insbesondere mit 5 bis 6 C-Atomen, Aralkyl, insbesondere mit 1 bis 4 C-Atomen im aliphatischen Teil, Aryl, insbesondere Phenyl, Halogen, Hydroxy, Alkoxy mit 1 bis 4 C-Atomen, Phenoxy, Carboxy, Alkoxycarbonyl mit 1 bis 4 C-Atomen, Nitro,
wobei
$R^1$ und $R^2$, $R^3$ und $R^4$, $R^5$ und $R^6$ weiterhin die Glieder zur Vervollständigung eines insbesondere 6-gliedrigen carbocyclischen Ringes gemeinsam mit dem Benzolkern darstellen können,
oder wobei jeweils einer der Reste $R^1$ bis $R^6$ Alkylen mit 1 bis 6 C-Atomen, Alkyliden mit 1 bis 4 C-Atomen, Phenylen, Phenalkylen oder Phenalkyliden darstellen kann und 2 Reste der Formel II miteinander verknüpft,
Z Wasserstoff, Alkyl mit 1 bis 6 C-Atomen oder

$$\frac{M^{z+}}{z}$$

M z-wertiges Metallkation eines Übergangsmetalls oder eines Metalls der 2. oder 3. Hauptgruppe, insbesondere wie Zn, Fe, Co, Ni, Cr, Mn, Cu, Mg, Ca, B, Al, Ti, Si, insbesondere Zn,
m eine ganze Zahl von 0 bis 10,
n eine ganze Zahl von 1 bis 4, wobei
m + n mindestens 2 bedeuten,
z 1 bis 4, besonders bevorzugt 2 oder 3.

Gegenstand der Erfindung ist weiterhin ein Verfahren zur Herstellung von oligobenzylierten aromatischen Hydroxycarbonsäuren durch Umsetzung einer Hydroxycarbonsäure mit Benzylhalogeniden in Gegenwart eines sauren Katalysators, dadurch gekennzeichnet, daß man die Hydroxycarbonsäure vorlegt und Benzylhalogenid allmählich nach Maßgabe zugibt, daß stets ein Konzentrationsverhältnis von aromatischer Hydroxycarbonsäure zu Benzylhalogenid von $\geq 1$ vorliegt, bei einer Temperatur, bei der eine Decarboxylierung noch nicht oder jedenfalls nicht im wesentlichen Umfange auftritt unter Mitverwendung von insbesondere farbaufhellenden Additiven. Die Umsetzung kann in Gegenwart von bi-oder trifunktionellen Verzweigern durchgeführt werden.

Gegenstand der Erfindung sind weiterhin Verbindungen erhältlich nach dem erfindungsgemäßen Verfahren.

Weiterhin wurde gefunden. daß die oligobenzylierten aromatischen Hydroxycarbonsäuren der allgemeinen Formel, ihre Ester, bevorzugt deren Metallsalze mit Übergangsmetallen oder mit Metallen der 2. und 3. Hauptgruppe des Periodensystens, wie Zn, Fe, Co, Ni, Cr, Mn, Cu, Mg, Co, B, Al, Ti, Si, insbesondere mit Zn, ausgezeichnete Farb entwickler mit hohen Farbintensitäten bei druck-oder wärmeempfindlichen Reaktionsaufzeichnungssystemen darstellen.

Aromatische Hydroxycarbonsäuren, die sich als Ausgangsprodukte eignen, sind beispielsweise Salicylsäure, Salicylsäuremethyl-, ethyl-und butylester, p-Hydroxybenzoesäure, p-Hydroxybenzoesäuremethyl-und ethylester, Methylsalicylsäure, tert.-Butylsalicylsäure, tert.-Octylsalicylsäure, Nonylsalicylsäure, Methyl-tert.-amylsalicylsäure, Cyclohexylsalicylsäure, Phenylsalicylsäure, Chlorsalicylsäure, 2,4-Dihydroxybenzoesäure, 3,5-Dihydroxybenzoesäure, 1-Hydroxynaphthalin-2-carbonsäure, 3-Hydroxynaphthalin-2-carbonsäure, 4,4'-Dihydroxy-diphenylmethan-dicarbonsäure, 5,5'-Phenylmethylen-bis-(2-hydroxy-3-methyl-benzoesäure), insbesondere Salicylsäure.

An Benzylhalogeniden kommen als Ausgangsprodukte beispielhaft in Frage Benzylchlorid. Benzylbromid, o-, m-, p-Methylbenzylchlorid, tert.-Butylbenzylchlorid, Dodecylbenzylchlorid, Chlorbenzylchlorid, m-Chlor-p-methylbenzylchorid, Chlormethyldiphenylether, Chlormethyldiphenyl und Chlormethylnaphthalin.

Die Benzylhalogenide aber auch die Hydroxybenzoesäuren können jeweils einzeln oder als Gemische untereinander eingesetzt werden. Die Menge an einkondensierbarem Benzylhalogenid ist nach oben nicht begrenzt. doch nimmt in der Regel mit steigender Menge die Farbentwicklerintensität ab. Im allgemeinen werden 2 bis 25 Mol, vorzugsweise ca. 2 bis 7 Mol pro Mol Hydroxybenzoesäure eingesetzt. Als Verzweiger zur Herstellung bimolekularer Verbindungen können auch bifunktionelle Benzylhalogenide, beispielsweise Benzylchlorid, Benzotrichlorid oder o-, m-, p-Xylylenhalogenide, bevorzugt -chloride eingesetzt werden. Die Mengen betragen in der Regel 1 bis 50 %, bevorzugt 5 bis 10 % der Menge an eingesetztem Benzylhalogenid. Die Polykondensation verläuft zu einem Gemisch oligobenzylierter Hydroxybenzoesäuren mit statistischer Verteilung.

Als saure Katalysatoren eignen sich sowohl Brönsted-Säuren, wie Phosphorsäure, Schwefelsäure und p-Toluolsulfonsäure, als auch Lewis-Säuren, wie $AlCl_3$, $BF_3$, $SnCL_4$, $SnCl_2$, $SbCl_5$, $TiCl_4$, $FeCl_3$, $ZnCl_2$, Zn-Acetat, Zn-Stearat, $CaCl_2$, $MgCl_2$, aber auch aktivierte Tonerden.

Die Katalysatorkonzentrationen können in weiten Grenzen variiert werden. Die Verwendung katalytischer oder stöchiometrischer Mengen an Säure richtet sich danach, ob man die erfindungsgemäßen oligobenzylierten aromatischen Hydroxycarbonsäuren mit Z = H oder Niederalkyl als freie Säuren oder Ester oder mit Z = Metall als Metallsalze anstrebt. In den Fällen, wo die freien Säuren bzw. deren Ester gewünscht werden, wird man katalytische Mengen an Brönsted-oder Lewis-Säuren, im allgemeinen von 0,01-10 %, bevorzugt von 0,1-2 % einsetzen.

In den Fällen, wo man das Metallsalz anstrebt, wird man die Kondensationsreaktion in Gegenwart von stöchiometrischen Mengen der entsprechenden Metall-Lewissäure durchführen, wobei in der Regel 1 Äquivalent pro Mol an Hydroxybenzoesäure zum Einsatz kommen.

Die Reaktionstemperaturen richten sich jeweils nach den Bedingungen, unter denen eine ausreichende Abspaltung von Halogenwasserstoff aus der Kondensationsreaktion abläuft und noch keine Decarboxylierung stattfindet. Sie liegen im allgemeinen zwischen ca. 50 bis 170°C, insbesondere zwischen ca. 80 und 130°C.

Die Umsetzung wird im allgemeinen in der Schmelze der Reaktanten durchgeführt. Bei Bedarf können jedoch auch Lösungsvermittler mitverwendet werden oder die Reaktion kann in einem Lösungsmittel ablaufen.

Als Lösungsvermittler kommen beispielsweise in Frage: Carbonsäuren, wie Ameisensäure, Essigsäure, Propionsäure, Bernsteinsäure, Adipinsäure, Geeignete Lösungsmittel sind z.B. Kohlenwasserstoffe, wie Tetrachlorkohlenstoff, Chlorbenzol, Dichlorbenzol, Trichlorbenzol, Trichlorethylen.

Für die erfindungsgemäße Verwendung als Farbentwickler für Reaktionsaufzeichnungssysteme ist es von Auschlag gebender Bedeutung, daß die erfindungsgemäßen Harze eine gute Farbqualität aufweisen. Zu diesem Zweck werden der Kondensationsreaktion vorzugsweise Additive zugesetzt, die erfindungsgemäß überraschend eine Farbaufhellung bewirken. Geeignete farbverbessernde Additive sind z.B. reduzierende Agenzien, wie Zn-Staub, $SnCl_2$, $TiCl_3$, Formaldehyd - gegebenenfalls in Kombination mit Ameisensäure - oder insbesondere $H_2O$. Es ist als überaus überraschend anzusehen. daß die erfindungsgemäße Halogenwasserstoff abspaltende Kondensationsreaktion auch in Gegenwart von Wasser abläuft und daß die Bildung von stark braunroten Nebenprodukten unterbleibt.

In einer allgemeinen Ausführungsform erhitzt man die aromatiscne Hydroxycarbonsäure, gegebenenfalls in einem inerten Lösungsmittel oder zusammen mit einem Lösungsvermittler, unter Zusatz eines farbverbessernden Additivs, in der Regel unter einem Schutzgas, wie Stickstoff, in Gegenwart katalytischer oder stöchiometrischer Mengen einer starken Säure oder eines Metallsalzes, gegebenenfalls zusammen mit einer unterschüssigen Menge des Benzylhalogenids auf Temperaturen, wo die Abspaltung von Halogenwasserstoff einsetzt und läßt die Hauptmenge an Benzylhalogenid allmählich bei Reaktionstemperatur einlaufen. Nach Beendigung der Halogenwasserstoffentwicklung entfernt man flüchtige Komponenten, wie Additive und Lösungsmittel, im Vakuum.

Das so hergestellte Harz kann auf dieser Stufe durch Zugabe eines Lösungsmittels und Wasser gewaschen werden. Restspuren an saurem Katalysator beeinflussen die Farbentwicklungseigenschaftn der Produkte nur unmerklich, so daß die Wäsche mit Wasser oft überflüssig ist.

In einer weiteren Ausführungsform wird die Kondensation zunächst nach der katalytischen Variante durchgeführt, dann die freie Säure der Formel II (Z = H) oder deren Niederalkylester (Z = Niederalkyl) nach Verseifung mit z.B. verdünnter Natronlauge mit der äquivalenten Menge einer metallabgebenden Verbindung in das gewünschte Metallsalz (Z = M) übergeführt. Die Herstellung von Metallsalzen aus Hydroxysäuren ist bekannter Stand der Technik.

Metallabgebende Verbindungen sind beispielsweise Metallhydroxide, - oxide, -alkoholate oder - carbonate, Metallsalze schwacher Säuren, wie Acetate oder Propionate oder Metallsalze mittelstarker oder starker Säuren in Gegenwart von Alkali-oder Erdalkalihydroxiden, -oxiden oder -carbonaten; beispielsweise $Zn(OH)_2$, ZnO. Zn-acetat, $ZnCl_2$, $ZnSO_4$, $Zn(NO_3)_2$, $Zn(OC_2H_5)_2$, $ZnCO_3$, $FeBr_3$, $FeCl_3$, $Fe(NO_3)_3 \bullet 9H_2O$, $Fe_2(SO_4)_3$, Fe-acetat, $AlBr_3$, $AlCl_3$, $Al(OH)_3$, $Al(NO_3)_3 \bullet H_2O$, $Al_2(SO_4)_3$, $CoCl_2$, $Co(OH)_2$, $Co(NO_3)_2 \bullet 6H_2O$, $CoSO_4 \bullet 7H_2O$, $NiBr_2$, $NiCl_2$, $NiCO_3$, $Ni(OH)_2$, $NiSO_4$, CaO, $Ca(OH)_2$, $CaCl_2$, $Ca(NO_3)_2 \bullet 4H_2O$, $CaSO_4$, MgO, $Mg(OH)_2$, $MgCO_3$, $MgCl_2$, $Mg(NO_3)_2 \bullet 6H_2O$, $MgSO_4$, $PbCl_2$, $Pb(NO_3)_2$, $PbSO_4$, $BCl_3$, $BF_3$, $TiCl_3$, $Ti_2(SO_4)_3$, $Ti(SO_4)_2$, $TiOSO_4$, $SnBr_2$, $SnCl_2$, $SnSO_4$, $CuCl_2$, $CuSO_4 \bullet 5H_2O$, $CrCl_2$, $Cr(NO_3)_3 \bullet 9H_2O$.

Die erfindungsgemäßen Produkte stellen vorzugsweise fast farblose bis blaßbeige Harze mit Erweichungspunkten von ca. 25 bis 125°C dar, und besitzen mittlere Molekulargewichte von 300 bis 3000, vorzugsweise ca. 400 bis 1000.

Die erfindungsgemäßen Harze können nach bekannten Verfahren in der Regel unter verwendung von Dispergiermitteln zu wäßrigen Dispersionen verarbeiten werden. In einer besonders geeigneten Verfahrensvariante werden die erfindungsgemäßen oligobenzylierten Hydroxysäuren der allgemeinen Formel II als freie Säuren (Z=H) in eine wäßrige Dispersion überführt und in dieser Form mit einer metallabgebenden Verbindung zu dem entsprechenden Metallsalz (Z=Metallkation) als stabile wäßrioge Dispersion umgesetzt.

Die erfindungsgemäßen Verbindungen können, gegebenenfalls in Form ihrer wäßrigen Dispersionen, als Farbentwickler beispielsweise zur Herstellung kohlefreier Durchschreibepapiere sowie zur Herstellung von thermoreaktiven Aufzeichnungsmaterialien verwendet werden. Hierzu werden die Dispersionen der erfindungsemäßen Farbentwickler auf eine Papierträgerbahn aufgestrichen. Die Formulierung derartiger Streichfarben ist bekannt.

Eine zur Herstellung eines kohlefreien Durchschreibepapiers zu verwendende Streichfarbe kann beispielsweise durch mechanische Dispergierung des Harzpulvers in Wasser erfolgen, das genügende Mengen an Dispergiermittel enthält.

Als Dispergiermittel können z.B. Polyvinylacetate, Polyvinylalkohole, Hydroxyethylcellulose, Gummiarabicum, Guar gum, Locust bean gum oder Ghatti gum verwendet werden. Besonders bevorzugt sind Dispersionen, die neben den erfindungsgemäßen Farbentwicklern Kombinationen aus verschiedenen Polysacchariden enthalten: Gummi arabicum und Guar gum, Gummi arabicum und Locust bean gum; hierbei wirkt Gummi arabicum offenbar als Dispergiermittel, während das andere Polysaccharid als Verdicker das Sedimetieren und die Agglomeration von Harzteilchen verhindert.

Zur mechanischen Dispergierung sind handelsübliche Kolloidmühlen, Perlmühlen, Kugelmühlen und ähnliche Homogenisiereinrichtungen geeignet.

Zur weitern Formulierung der Streichfarben werden der wäßrigen Dispersion häufig Absorbentien wie Kreide, Steichclay, Aluminiumsilikate etc. zugegeben.

Ferner können die erfindungsgemäßen Verbindungen, als "Hybrid-Systeme" eingesetzt werden, d.h. sie können etwa mit chemisch modifizierten Aluminium-Schichtsilikaten auf Montmorrillonit-Basis kombiniert werden.

Weiterhin müssen die Streichfarben mit Binder versehen werden, um die Beschichtungsmasse auf einen Träger zu fixieren. Da bevorzugt Papier als Trägerstoff in Frage kommt, handelt es sich bei diesen Bindern hauptsächlich um Papierbeschichtungsmittel, wie Gummiarabikum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylmono-oder - copolymere.

Neben der Verwendung in wäßrigen Streichfarben ist jedoch auch die Einarbeitung in Druckfarben für Flexo-oder Offsetdruck möglich.

Bei der Herstellung einer Offset-oder Buchdruckfarbe können die erfindungsgemäßen Entwicklerharze mit einem geeigneten Firnis auf einem Dreiwalzenstuhl angerieben werden. Die Herstellung derartiger Offsetdruckfarben ist bekannter Stand der Technik.

Flexodruckfarben enthalten neben Bindemitteln ein leicht siedendes Lösungsmittel. Hierfür sind als Lösungsmittel z.B. $C_1$-$C_6$-Alkohole, $C_2$-$C_4$-Alkandiole, -triole, Ethylenglykolmonoalkylether, aromatische und/oder chlorierte Kohlenwasserstoffe, Ester und/oder Ketone geeignet. Lösungsmittel sind ferner Methanol, Ethanol, n-Propanol, Isopropanol, n-Butanol, Isobutanol, sec.-Butanol, Ethylenglykolmonomethylether, Ethylenglykol, 1,2-Propandiol, Glycerin, Aceton, Methylethylketon, Toluol, Xylol, Methylenchlorid, Trichlorethan oder Gemische davon. Die Herstellung von Flexodruckfarben gehört ebenfalls zum längst bekannten Stand der Technik.

Mit Hilfe der Druckfarben lassen sich bestimmte Zonen des Trägermaterials spotweise beispielsweise mit Offset-oder Flexodruckwerken auftragen.

Arbeitet man nach diesem Verfahren, können spotbeschichtete Entwicklerpapiere hergestellt werden und das bisher übliche, zonenweise Neutralisieren eines ganzflächig beschichteten Entwicklerpapiers and den Stellen, wo aus Sicherheitsgründen keine Durchschrift erzeugt werden soll, kann entfallen.

Die erfindungsgemäßen Verbindungen können ebenfalls zur Herstellung von Thermopapieren verwendet werden.

Thermoreaktive Aufzeichnungssysteme umfassen z.B. wärmeempfindliche Aufzeichnungs-und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Meßinstrumenten verwendet, wie z.B. Elektrocardiographen. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, daß Farbbildner in einer Bindemittelschicht gelöst oder dispergiert ist und in einer zweiten Schicht der erfindungsgemäße Entwickler in dem Bindemittel gelöst oder dispergiert ist. Eine andere Möglichkeit besteht darin, daß sowohl die Farbbildner als auch der erfindungsgemäße Farbentwickler in einer Schicht dispergiert sind. Der Farbentwickler wird in spezifischen Bezirken mittels Wärme erweicht und an diesen Punkten, an denen Wärme angewendet wird, kommt der Farbbildner mit dem erfindungsgemäßen Benzylharz in Kontakt und es entwickelt sich sofort die erwünschte Farbe.

Die Herstellung von Thermopapieren gehört zum bekannten Stand der Technik. In den folgenden Herstellungs-und Anwendungsbeispielen beziehen sich die angegebenen Prozentsätze, wenn nichts anderes angegeben ist, auf das Gewicht und Teile sind Gewichtsteile.

<u>Beispiel 1</u>

1038 g (7,5 Mol) Salicylsäure, 1012,8 g (8 Mol) Benzyl-chlorid, 63,2 g (0,5 Mol) $ZnCl_2$ und 50 ml $H_2O$ werden unter Rühren und Druchleiten von Stickstoff in einem Ölbad aufgeschmolzen, wobei bei 120°C eine kräftige Abspaltung von HCl einsetzt. Innerhalb von 3 Stunden läßt man noch weitere 2785,2 g (22 Mol) Benzylchlorid bei 120 bis 130°C zulaufen und rührt noch 5 Stunden unter Stickstoffdurchleitung durch die Schmelze bis zur Beendigung der HCl-Entwicklung bei gleicher Temperatur nach. Das anschließende Abziehen flüchtiger Komponenten im Vakuum liefert nur geringfügige Mengen (ca. 10 g) Destillat. Die Ausbeute beträgt 3789 g (99,9 % der Theorie) eines hellen gelblich bräunlichen spröden Harzes.

OH-Zahl 28; Säurezahl 115

Viskosität

$\pi$ 128°C 102,9 cP

$\pi$ 107°C 374,9 cP

$\pi$ 93°C 1327,6 cP

Die $ZnCl_2$-freie Substanz läßt sich leicht durch Waschen des Harzes mit z.B. 1n Salzsäure und dann mit Wasser, gegebenenfalls unter Zuhilfenahme eines Lösungsmitels wie Toluol oder Mehtylenchlorid gewinnen.

OH-Zahl 38,5°; Säurezahl 108,5

Beispiel 2

Gemäß der Verfahrensweise von Beispiel 1 erhält man bei der Kondensation von 553.6 g (4 Mol) Salicylsäure mit insgesamt 1519,2 g (12 Mol) Benzylchlorid in Gegenwart von 21,8 g (0,16 Mol) $ZnCl_2$ und 15 g $H_2O$, 1640 g (99,0 % der Theorie) eines leicht beige bräunlichen hochviskosen Harzes.

Beispiel 3

Die Kondensation gemäß Beispiel 1 von 553,6 g (4 Mol) Salicylsäure mit insgesamt 1012,8 g (8 Mol) Benzylchlorid in Gegenwart von 21,8 g (0,16 Mol) $ZnCl_2$ und 15 ml Wasser leifert 1284,3 g (99,2 %) eines hell gelblichen geruchslosen viskosen klebrigen Harzes.

Beispiel 4

Eine Mischung aus 34,6 g (0,25 Mol) p-Hydroxybenzoesäure 1,36 g (0,01 Mol) $ZnCl_2$, 63,3 g (0,5 Mol) Benzylchlorid und 2 ml $H_2O$ wird vorsichtig im Ölbad unter Stickstoff bei 140 bis 150°C aufgeschmolzen, dann läßt man weitere 63,3 g (0,5 Mol) Benzylchlorid im Verlaufe von 1 1/2 Stunden bei 130 bis 140°C in die Schmelze eintropfen. Zur Vervollständigung der HCl-Abspaltung leitet man noch 5 Stunden Stickstoff bei derselben Temperatur durch die Schmelze, zieht im Verlauf von ca. 0,5 Stunden bei ca. 120 bis 140°C im Vakuum flüchtige Komponenten (1,5 g) ab und erhält auf diese Weise 119,7 g (96 % der Theorie) eines leicht orange gefärbten spröden pulverbaren Harzes vom Erweichungspunkt 44 bis 56°C.

Beispiel 5

Zu 304,3 g (2 Mol) Salicylsäuremethylester und 0,5 g Zn-Stearat läßt man unter Rühren 1012,8 g (8 Mol) Benzylchlorid bei 120°C innerhalb 2 Stunden eintropfen, wobei ein kräftiger Strom von HCl entweicht. Man hält noch ca. 3 Stunden unter Durchleiten von trockenem Stickstoff durch die Schmelze bei 130°C, destilliert zum Schluß gegebenenfalls geringfügige Mengen (ca. 7 g) flüchtigen Materials im Vakuum bei ca. 130°C ab und erhält auf diese Weise 1011,6 g (98,7 % der Theorie) eines bräunlich beige gefärbten viskosen klebrigen Harzes.

Ber. C 84,35% H 6,29% O 9,36%
Gef. 84,54% 6,30% 9,18

Beispiel 6

Analog Beispiel 5 erhält man aus 152,0 g (1 Mol) Salicylsäuremethylester, 0,25 g Zn-Stearat und 379,8 g (3 Mol) Benzylchlorid 411 g (97,3 %) eines bräunlich viskosen klebrigen Harzes.

Ber. C 82,44% H 6,20% O 11,36%
Gef. 82,60 6,13 11,20

Beispiel 7

In der gleichen Weise, wie in Beispiel 5 beschrieben, werden 152,0 g (1 Mol) Salicylsäuremethylester, 0,25 g Zn-Stearat und 253,2 g (2 Mol) Benzylchorid zum "Dibenzyl"-salicylsäuremethylester umgesetzt. 325 g (97,8 %) bräunlich transparentes viskoses Öl.

Ber. C 79,49% H 6,07 O 14,44%
Gef. 79,68 6,17 14,28

Beispiel 8

152 g (1 Mol) p-Hydroxybenzoesäuremethylester, 1 g Zn-Stearat und 126.6 g (1 Mol) Benzylchlorid werden unter Stickstoff aufgeschmolzen, dann weitere 379,8 g (3 Mol) Benzylchorid im Verlauf von 3 Stunden bei 110 bis 130°C eingetropft. Anschließend vervollständigt man die HCl-Abspaltung durch mehrstündiges Druchleiten von trockenem Stickstoff durch die Schmelze bei ca. 130°C, zieht gegebenenfalls Restmengen flüchtigen Materials im Vakuum bei ca. 150°C ab (10 g öliges Destillat) und erhält 495 g (96,6 % der Theorie) eines bräunlichen spröden Harzes.

Ber. C 84,35% H 6,29% O 9,36%

Gef. 84,40 6,30 9,45%

Beispiel 9

In das gemäß Beispiel 1 hergestellte Harz (3789 g, ca. 7,5 Mol) rührt man beim Abkühlen der Schmelze bei ca. 80°C ca. 500 ml Toluol ein, fügt 735 g (ca. 8 Mol) an 45 %iger Natronlauge, danach eine Lösung von 410,5 g (3.25 Mol) $ZnCl_2$ in 1500 ml $H_2O$ zu und rührt noch ca. 30 min bei 80°C nach, bis sich 2 klare Phasen ausgebildet haben. Die wäßrige NaCl-Lösung wird abgetrennt, die organische einmal mit ca. 500 ml $H_2O$ gewaschen und eingeengt. Nach Trocknen im Vakuum bei ca. 100°C erhält man ein sprödes Harz von EP 108-124°C, das sich leicht zu einem hellen gelblich-beige gefärbten Pulver mahlen läßt; die Ausbeute beträgt 3975 g.

Beispiel 10

In das gemäß Beispiel 1 hergestellte Harz (3789 g, ca. 7,5 Mol) läßt man beim Abkühlen der Schmelze bei ca. 100°C unter gutem Rühren mit einem Ankerrührer 1990 g einer 10 %igen wäßrigen Lösung eines teilverseiften Polyvinylacetates (Moviol® 8-88 der Fa. Hoechst) sowie 135 g (ca. 1,5 Mol) 45 %iger Natronlauge einlaufen und rührt noch 30 min bei 60-70°C nach, bis sich eine stabile farblose Dispersion ausgebildet hat. In diese rührt man eine glatte Aufschlämmung von 265 g (3,25 Mol) ZnO in 1780 g $H_2O$ allmählich in dem Maße ein, daß sich eine Temperatur von ca. 40-45°C einstellt. Man hält noch ca. 1 h unter gutem Rühren bei dieser Temperatur, bis sich der Zn-Komplex voll ausgebildet hat. Zur Entfernung eventueller Stippen walzt man über einen Dreivalzenstuhl ab und erhält 7955 g einer fast farblosen viskosen aber gießbaren Dispersion. Viskosität (nach Brookfield, Spindel 7/100U): 10400 cP.

Beispiel 11

In das gemäß Beispiel 1 hergestellt Harz (3789 g, ca. 7,5 Mol) läßt man nach Abkühlen der Schmelze bei ca. 100°C 3500 ml Xylol unter Rühren einlaufen, fügt 713,3 g (3,25 Mol) $Zn(Ac)_2 \bullet 2H_2O$ zu und destilliert Wasser und Essigsäure - zunächst azeotrop - ab. Nach weiterem Einengen, zum Schluß im Strahlervakuum bei ca. 100°C erhält man das "Tetrabenzyl"-Zn-Salicylat als sprödes hellbeige gefärbtes leicht pulverbares Harz; die Ausbeute beträgt 3973 g, der EP 100 - 118°C.

Beispiel 12

In das gemäß Beispiel 1 hergestellte Harz (3789 g, ca 7,5 Mol) rührt man nach Abkühlen der Schmelze bei ca. 80°C ca. 5000 ml Toluol, dann 100 g konzentrierte Salzsäure in 1000 ml Wasser ein, trennt die wäßrige Phase ab und wäscht die organische mit ca. 1000 ml Wasser nach. Zu der Zn-freien toluolischen Lösung fügt man sodann 219 g (3,75 Mol) $Mg(OH)_2$ zu und rührt ca. 30 min bis zur Ausbildung von 2 klaren Phasen bei 80°C, destilliert Wasser azeotrop über einen Abscheider ab, filtert die Toluollösung und engt weiter, zum Schluß im Vakuum bei ca. 100°C, ein. Nach Mahlen des hellen spröden Harzes über eine Pulvermühle erhält man 3812 g eines leicht cremefarbenen Pulvers von EP 100 - 110°C.

Beispiel 13

138.1 g (1 Mol) Salicylsäure werden zusammen mit 68,2 g (0,5 Mol) $ZnCl_2$ und 60 ml Essigsäure unter Rühren und unter Stickstoff aufgeschmolzen. bei ca. 100°C fügt man erst 1 g Paraformaldehyd, dann 60 ml Ameisensäure zu and läßt dann innerhalb 2 h 569,7 g (4,5 Mol) Benzylchorid bei 100 bis 115°C einlaufen. Anschließend hält man 5 Stunden unter Durchleiten von Stickstoff durch die Schmelze bei 150°C, zieht geringfügige Mengen flüchtigen Materials bei der gleichen Temperatur im Vakuum ab und erhält 573, 2 g (99,6 %) eines orange gelblichen spröden Harzes.

Analog der Verfahrensweise von Beispiel 13 wurden weitere aromatische Hydroxycarbonsäuren mit Benzylchlorid umgestzt (siehe Beispiel 14-17) sowie weitere Benzylchloride mit 1 Mol Salicylsäure umgesetzt (siehe Beispiele 18 - 21).

| Beispiel | Ausgangssäure | Benzylchlorid | Harz (Zn-Salz) |
|---|---|---|---|
| 14 | 188 g (1 Mol) 1-Hydroxy-naphthalin-2-carbonsäure | 633 g (5 Mol) | 666,1 g (99,4 %) bräunlichbeige, spröd |
| 15 | 194 g (1 Mol) 5-tert.-Butyl-salicylsäure | 379,8 g (3 Mol) | 494,4 g (99,7 %) orangebeige, spröd |
| 16 | 152 g (1 Mol) 3-Methyl-salicylsäure | 759,6 g 6 Mol) | 715,5 g (98,8 %) cremefarben, spröd |
| 17 | 144 g (0,5 Mol) 4,4'-Di-hydroxydiphenylmethan-dicarbonsäure (3,3') | 379,8 g (3 Mol) | 438,9 g (98,4 %) bräunlich beige, spröd |

| Beispiel | subst. Benzylchlorid | Harz (Zn-Salz) |
|---|---|---|
| 18 | 644 g (4 Mol) p-Chlorbenzylchlorid | 681 g (99,3 %) hellbeige, spröd |
| 19 | 844 g (6 Mol) p-Methylbenzylchlorid | 787 g (99 %) hellbräunlich, spröd |
| 20 | 883,5 g (5 Mol) 1-Chlormethylnaphthalin | 853,6 g (98 %) gelblich beige, spröd |
| 21 | { 506,4 g (4 Mol) Benzylchlorid + 70 g (0,4 Mol) p-Xylylenchlorid } | 588,8 g (96,9 %) orange gelblich, spröd |

Beispiel 22 Anwendungsbeispiel (kohlefreie Durchschreibepapiere)

3.1 l Wasser werden mit Natronlauge auf pH 9,0 gestellt und 1 kg China Clay SPS eingerührt. Unter weiterem Rühren werden 500 g einer 50 %igen Harzemulsion, hergestellt gemäß Beispiel 10, zugegeben. Anschließend erfolgte die Zugabe von 200 g einer 5 %igen wäßrigen CMC-Lösung und von 160 g eines 50 %igen SBR-Latex-Binders.

Die so hergestellte Streichfarbe wurde mit einer Luftbürstenbeschichtungsanlage auf ein Rohpapier für kohlefreie Durchschreibepapiere von 45 g/m² aufgestrichen. Der Auftrag betrug trocken ca. 5 g/m² Papier.

Legt man ein handelsübliches CB-Blatt (z.B. Autocopy der Fa. Zanders) auf die beschichtete Seite des so erhaltenen Entwicklerblattes und beschreibt das Oberblatt, so erhält man eine intensive Kopie auf dem Entwicklerblatt, die selbst bei längerer Lichteinwirkung nur wenig und bei Feuchteeinwirkung nicht verblaßt.

Beispiel 23 Anwendungsbeispiel (Thermopapier)

In einer Kugelmühle werden 32 g des Harzes gemäß Beispiel 9, 3,8 g Distearylamid des Ethylendiamins, 89 g Kaolin, 20 g eines zu 88 % hydrolysierten Polyvinylalkohols und 55 ml Wasser gemahlen, bis die Teilchengröße ca. 5 µm beträgt. In einer zweiten Kugelmühle werden 6 g Kristallviolettlacton, 3 g eines zu 88 % hydrolysierten Polyvinylalkohols und 60 ml Wasser zu einer Teilchengröße von ca. 3 µm gemahlen. Die beiden Dispersionen werden zusammengegeben und mit einem Trockenauftragsgewicht von 5,5 g/m² auf Papier gestrichen. Durch Erhitzen des Papiers auf 120°C wird eine intensive blaue Farbe erhalten, die eine gute Licht-und Sublimierechtheit hat.

Beispiel 24 Anwendungsbeispiel

250 Tle. Harz des Beispiels 13 werden zusammen mit 50 Tln. Teilverseiften Polyvinylacetates (Moviol® 8-88 der Firma Hoechst) und 200 Tln. Wasser in einer Kugelmühle gemahlen, bis eine 50 % Harz enthaltende, feinteilige (ca. 5µm) Dispersion erhalten wurde.

Die Dispersion wurde gemäß Beispiel 22 zu einer Streichfarbe verarbeitet und auf ein Rohpapier aufgestrichen.

Legt man ein handelsübliches CB-Blatt (z. B. Autocopy der Fa. Zanders) auf die beschichtete Seite und beschreibt das Oberblatt, erhält man eine intensive, feuchtigkeitsstabile Kopie.

Beispiel 25

Das Harz des Beispiels 12 wird, wie im Beispiel 24 beschrieben, zu einem kohlefreien Durchschreibepapier verarbeitetf.

Man erhält ein analoges Ergebnis.

## Ansprüche

1. Verbindungen folgender Formel I

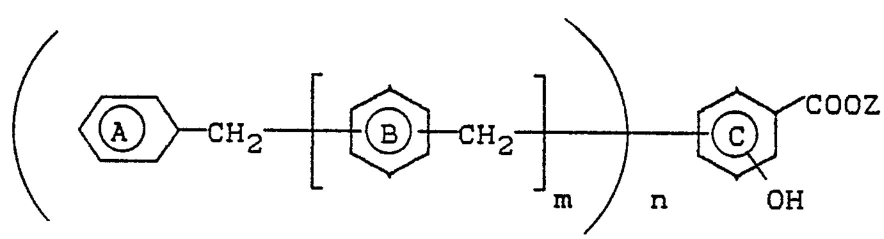

I

worin bedeuten

Z Wasserstoff, Alkyl mit 1 bis 6 C-Atomen oder

$$\frac{M^{z+}}{z}$$

M Metallkation
z 1 bis 4
n 1 bis 4
m 0 bis 20

mit der Maßgabe, daß m + n wenigstens 2 bedeuten, und wobei die Ringe A, B und C unabhängig voneinander unsubstituiert sein können oder substi tuiert sein können und wobei die Verbindungen auch bimolekular vorliegen können indem ein Substituent 2 Reste der Formel I miteinander verknüpft.

2. Verbindungen gemäß Anspruch 1, dadurch gekennzeichnet, daß M = Zn, Fe, Co, Ni, Cr, Mn, Cu, Mg, Ca, B, Al, Ti, Si bedeutet.

3. Harze, die im wesentlichen Umsetzungsprodukte einer aromatischen Hydroxycarbonsäure und von Benzylhalogeniden enthalten, dadurch gekennzeichnet, daß das Umsetzungsprodukt der Formel (I) entspricht.

4. Verbindungen erhältlich durch Umsetzung von aromatischen Hydroxycarbonsäure mit Benzylhalogeniden in Gegenwart eines sauren Katalysators, dadurch gekennzeichnet, daß man die Hydroxycarbonsäure vorlegt und Benzylhalogenid allmählich zugibt bei einer Temperatur, bei der eine Decarboxylierung nicht oder im wesentlichen nicht auftritt.

5. Druck-oder wärmeempfindliches Reaktionsaufzeichnungssystem, dadurch gekennzeichnet, daß es eine Verbindung gemäß wenigstens einem der vorhergehenden Ansprüche enthält.

6. Aufzeichnungssytem gemäß Anspruch 5, dadurch gekennzeichnet, daß die Verbindung ein Metallsalz ist.

7. Aufzeichnungssystem nach Anspruch 5, dadurch gekennzeichnet, daß die Verbindung als Farbentwickler enthalten ist.

8. Verfahren zur Herstellung von oligobenzylierten aromatischen Hydroxycarbonsäuren durch Umsetzung von aromatischen Hydroxycarbonsäuren durch Umsetzun von aromatischen Hydroxycarbonsäuren mit Benzylhalogeniden in Gegenwart eines sauren Katalysators, gegebenenfalls in Gegenwart eines Lösungsmittels, dadurch gekennzeichnet, daß man Benzylhalogenid allmählich in dem Maße zu der Hydroxysäure gibt, daß stets ein Konzentrationsverhältnis von aromatischer Hydroxycarbonsäure zu Benzylhalogenid von ≧ 1 vorliegt, bei einer Temperatur, bei der eine Decarboxylierung nicht oder im wesentlichen nicht auftritt.

9. Verfahren nach Anspruch 8, dadurch gekennzeichnet, daß man zur Reaktion farbaufhellende Additive zugibt.

10. Verfahren zur Herstellung von stabilen wäßrigen Dispersionen von Hydroxycarbonsäuren gemäß Anspruch 1 mit Z = $M^{z+}/z$, dadurch gekennzeichnet, daß man die freien Säuren (I, Z = H) dispergiert und mit metallabgebenden Verbindungen umgesetzt werden.